# FASCICULE DE BREVET EUROPEEN

(11) **EP 4 580 499 B1**
(45) Date de publication et mention de la délivrance du brevet: **04.11.2026**
(21) Numéro de dépôt: 23783467.6
(22) Date de dépôt: 24.08.2023
(51) Int. Cl.: A61B 5/353, A61B 5/361, A61B 5/367, A61B 5/00, A61B 5/287, A61B 5/282

(54) **DISPOSITIF DE TRAITEMENT DE SIGNAUX CARDIAQUES**
VORRICHTUNG ZUR VERARBEITUNG VON HERZSIGNALEN
DEVICE FOR PROCESSING CARDIAC SIGNALS

(30) Priorité: 02.09.2022 FR 2208851
(43) Date de publication de la demande: 09.07.2025
(73) Titulaire: Substrate HD, 13006 Marseille (FR)
(72) Inventeur: BOUDOU, Thomas, 13006 Marseille (FR); HAJJAR, Céline, 13006 Marseille (FR)
(74) Mandataire: Plasseraud IP
(86) Numéro de dépôt international: PCT/FR2023/051289
(87) Numéro de publication internationale: WO 2024/047307

(56) Documents cités:
- US-A1- 2002 026 220
- US-A1- 2022 015 711
- QIAN ZHI-YONG ET AL: "An Algorithm to Predict the Site of Origin of Focal Atrial Tachycardia : LOCALIZATION OF FOCAL ATRIAL TACHYCARDIA", PACE - PACING AND CLINICAL ELECTROPHYSIOLOGY., vol. 34, no. 4, 22 November 2010 (2010-11-22), US, pages 414 - 421, XP093031913, ISSN: 0147-8389, DOI: 10.1111/j.1540-8159.2010.02980.x
- KISTLER P M ET AL: "P-Wave Morphology in Focal Atrial Tachycardia", JOURNAL OF THE AMERICAN COLLEGE OF CARDIOLOGY, ELSEVIER, AMSTERDAM, NL, vol. 48, no. 5, 5 September 2006 (2006-09-05), pages 1010 - 1017, XP025147425, ISSN: 0735-1097, [retrieved on 20060905], DOI: 10.1016/J.JACC.2006.03.058

## Description

L'invention concerne le domaine du traitement des signaux cardiaques. Plus précisément, elle trouve à s'appliquer dans le domaine du traitement de la fibrillation atriale.

Le traitement de la tachycardie atriale (ou "TA", ou *"atrial tachycardia"* en anglais) consiste en général à brûler la zone du cœur qui est à l'origine de la tachycardie. Pour cela, il faut d'abord détecter cette zone.

La plupart des articles de recherche qui donnent un aperçu du problème de localisation de la TA sont principalement basés sur l'analyse des "ondes P", une partie spécifique de l'électrocardiogramme (ci-après "ECG") qui correspond à la dépolarisation des oreillettes.

En 1995, Tang et al se sont intéressés dans l'article "Use of p wave configuration during atrial tachycardia to predict site of origin", Journal of the American College of Cardiology, 26(5):1315-1324, à l'analyse de la polarité des ondes P dans les électrodes de surface pour déterminer quelle oreillette (la droite ou la gauche) est à l'origine de la tachycardie. Les dérivations aVL et V1 se sont avérées être les plus utiles pour distinguer les foyers droits des foyers gauches : une onde P positive dans la dérivation aVL prédit un foyer droit avec une sensibilité de 88 % et une spécificité de 79 %. La sensibilité et la spécificité d'une onde P positive dans la dérivation V1 prédisant un foyer gauche étaient respectivement de 93 % et 88 %.

Depuis, plusieurs études ont étendu l'analyse de la polarité des ondes P pour prédire la localisation du foyer avec une plus grande précision. En 2006, Kistler et al, dans l'article "P-Wave Morphology in Focal Atrial Tachycardia: Development of an Algorithm to Predict the Anatomie Site of Origin", 48:1010-1017, ont étudié 130 TA focaux pour construire un arbre de décision afin de trouver l'origine parmi 11 régions possibles, où les nœuds sont divisés selon la polarité des ondes P dans les différentes dérivations ECG. Cet algorithme a réussi à classer correctement l'origine dans 93% des 30 nouveaux TA. Un autre algorithme est connu de Qian Zhi-Yong et al., "An Algorithm to Predict the Site of Origin of Focal Atrial Tachycardia: Localization of Focal Atrial Tachycardia", PACE - Pacing and Clinical Electrophysiology, vol. 34, no 4, 22 novembre 2010, p. 414-421, ISSN 0147-8389, DOI : 10.1111/j.1540-8159.2010.02980.x.

Cependant, les critères retenus dans ces articles empêchent de les utiliser dans un contexte temps réel. En effet, ils reposent sur un traitement a posteriori permettant de détecter les ondes P de manière très précise, ce qui n'est pas possible en temps réel. Sur la base d'ondes P déterminées en temps réel, les résultats seraient nettement moins probants et ces méthodes inadaptées.

L'invention vient améliorer la situation. À cet effet, elle propose un dispositif de traitement de signaux cardiaques comprenant une mémoire agencée pour recevoir des jeux de données d'entrée comprenant chacun une pluralité de segments d'onde P chacun associé à une piste d'électrocardiogramme et à une fenêtre temporelle d'acquisition, et une pluralité de signaux de sinus coronaire associés à la même fenêtre temporelle d'acquisition et présentant une ou plusieurs séquences d'activation, un extracteur agencé, pour un jeu de données d'entrée donné, pour déterminer pour certains au moins des segments d'onde P du jeu de données d'entrée donné un type de profil de polarité d'onde, au moins une caractéristique d'extremum d'un type choisi dans un groupe de types comprenant le nombre d'extrema locaux positifs, le nombre d'extrema locaux négatifs, le maximum de prominence positive et le maximum de prominence négative, et au moins une valeur d'intégrale de ces segments d'onde P, et pour associer les données résultantes en groupe de données d'onde P de piste en fonction de la piste d'électrocardiogramme à laquelle est associé chaque segment d'onde P à partir duquel lesdites données résultantes ont été calculées. L'extracteur est en outre agencé pour déterminer, pour chaque groupe de données d'onde P de piste, un ensemble de caractéristiques d'onde P de piste comprenant un type de profil de polarité, une valeur de caractéristique d'extremum de jeu de données pour chaque type de caractéristique d'extremum calculé et une valeur d'intégrale déterminés à partir des données du groupe de données d'onde P de piste correspondant. L'extracteur est en outre agencé pour déterminer des temps d'activation dans certaines au moins des séquences d'activation des signaux de sinus coronaire du jeu de données d'entrée donné et pour en tirer un jeu de valeurs de temps, et pour retourner un ensemble de caractéristiques de jeu de données comprenant d'une part le jeu de valeurs de temps, et d'autre part les ensembles de caractéristiques d'onde P de piste. Le dispositif comprend également un localisateur à base d'apprentissage automatique utilisant des arbres décisionnels agencé pour recevoir en entrée un ensemble de caractéristiques de jeu de données, et pour retourner en sortie un identifiant de région cardiaque.

Ce dispositif est particulièrement avantageux car il permet de déterminer en temps réel une zone probable de l'anomalie à l'origine de la perpétuation de la TA. Cela signifie qu'un praticien a la possibilité de se concentrer sur une partie réduite du cœur pour déterminer ce qui constitue à ses yeux la vraie zone à l'origine de la TA. La détermination de la zone probable d'origine de la TA permet ainsi au praticien de gagner un temps précieux, bien que celle-ci n'ait pas de sens médical affirmé ni la valeur d'un diagnostic.

L'invention est définie par les revendications annexées. Selon divers modes de réalisation, la présente divulgation peut présenter une ou plusieurs des caractéristiques suivantes :
- le localisateur est agencé pour mettre en œuvre un classificateur à forêts aléatoires,
- l'extracteur est agencé pour déterminer une valeur de caractéristique d'extremum de jeu de données indiquant une absence de détermination pour un type de caractéristique d'extremum non calculé, et pour retourner un ensemble de caractéristiques de jeu de données comprenant une valeur de caractéristique d'extremum de jeu de données pour chaque type de caractéristique d'extremum,
- l'extracteur est agencé pour déterminer une valeur de caractéristique d'extremum de jeu de données pour chaque type du groupe de types,
- l'extracteur est agencé, pour un ensemble de caractéristiques d'onde P de piste donné, pour déterminer le type de profil de polarité en retenant le type de profil de polarité d'onde majoritaire dans le groupe de données d'onde P de piste correspondant,
- l'extracteur est agencé, pour un ensemble de caractéristiques d'onde P de piste donné, pour déterminer la valeur de caractéristique d'extremum de jeu de données pour chaque type de caractéristique d'extremum calculé et la valeur d'intégrale à partir de la moyenne de ces valeurs dans le groupe de données d'onde P de piste correspondant,
- l'extracteur est agencé pour calculer le jeu de valeurs de temps à partir de la différence entre les temps d'activation des séquences d'activation des signaux de sinus coronaire, et
- le localisateur est agencé pour recevoir en entrée un ensemble de caractéristiques de jeu de données comprenant 9 ensembles de caractéristiques d'onde P de piste, et un jeu de valeurs de temps comprenant 4 valeurs.

L'invention concerne également un procédé de traitement de signaux cardiaques comprenant les opérations suivantes :
a) recevoir des jeux de données d'entrée comprenant chacun une pluralité de segments d'onde P chacun associé à une piste d'électrocardiogramme et à une fenêtre temporelle d'acquisition, et une pluralité de signaux de sinus coronaire associés à la même fenêtre temporelle d'acquisition et présentant une ou plusieurs séquences d'activation,
b) pour un jeu de données d'entrée donné, pour certains au moins des segments d'onde P du jeu de données d'entrée donné :
   b1) déterminer un type de profil de polarité d'onde,
   b2) déterminer au moins une caractéristique d'extremum d'un type choisi dans un groupe de types comprenant le nombre d'extrema locaux positifs, le nombre d'extrema locaux négatifs, le maximum de prominence positive et le maximum de prominence négative, et
   b3) déterminer au moins une valeur d'intégrale de ces segments d'onde P,
   b4) associer les données résultantes des opérations b1) à b3) en groupe de données d'onde P de piste en fonction de la piste d'électrocardiogramme à laquelle est associé chaque segment d'onde P à partir duquel lesdites données résultantes ont été calculées,
   b5) déterminer, pour chaque groupe de données d'onde P de piste, un ensemble de caractéristiques d'onde P de piste comprenant un type de profil de polarité, une valeur de caractéristique d'extremum de jeu de données pour chaque type de caractéristique d'extremum calculé et une valeur d'intégrale déterminés à partir des données du groupe de données d'onde P de piste correspondant,
   b6) déterminer des temps d'activation dans certaines au moins des séquences d'activation des signaux de sinus coronaire du jeu de données d'entrée donné et pour en tirer un jeu de valeurs de temps,
   b7) retourner un ensemble de caractéristiques de jeu de données comprenant d'une part le jeu de valeurs de temps de l'opération b6), et d'autre part les ensembles de caractéristiques d'onde P de piste de l'opération b5), et
c) fournir un ensemble de caractéristiques de jeu de données obtenu à l'opération b7) en entrée d'un localisateur à base d'apprentissage automatique utilisant des arbres décisionnels, et retourner en sortie un identifiant de région cardiaque.

Selon divers modes de réalisation, le procédé peut présenter une ou plusieurs des caractéristiques suivantes :
- l'opération b2) comprend déterminer une valeur de caractéristique d'extremum de jeu de données pour chaque type du groupe de types,
- l'opération b5) comprend, pour un ensemble de caractéristiques d'onde P de piste donné, déterminer le type de profil de polarité en retenant le type de profil de polarité d'onde majoritaire dans le groupe de données d'onde P de piste correspondant,
- l'opération b5) comprend, pour un ensemble de caractéristiques d'onde P de piste donné, déterminer la valeur de caractéristique d'extremum de jeu de données pour chaque type de caractéristique d'extremum calculé et la valeur d'intégrale à partir de la moyenne de ces valeurs dans le groupe de données d'onde P de piste correspondant, et
- l'opération b6) comprend calculer le jeu de valeurs de temps à partir de la différence entre les temps d'activation des séquences d'activation des signaux de sinus coronaire.

L'invention concerne également un programme informatique comprenant des instructions pour exécuter le procédé selon l'invention, un support de stockage de données sur lequel est enregistré un tel programme informatique et un système informatique comprenant un processeur couplé à une mémoire, la mémoire ayant enregistré un tel programme informatique.

D'autres caractéristiques et avantages de l'invention apparaîtront mieux à la lecture de la description qui suit, tirée d'exemples donnés à titre illustratif et non limitatif, tirés des dessins sur lesquels :
- la [Fig.1] représente un diagramme schématique d'un dispositif selon l'invention,
- la [Fig.2] représente un exemple de fonction mise en œuvre par le dispositif de la [Fig.1],
- la [Fig.3] représente un exemple de profils de polarité d'onde P recherchés dans la fonction de la [Fig.2],
- la [Fig.4] représente un exemple de segment d'onde P et les types de caractéristiques mesurées,
- la [Fig.5] représente un exemple de segments de sinus coronaires et les valeurs de temps qui en sont tirées, et
- la [Fig.6] représente une division des oreillettes en 21 zones.

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

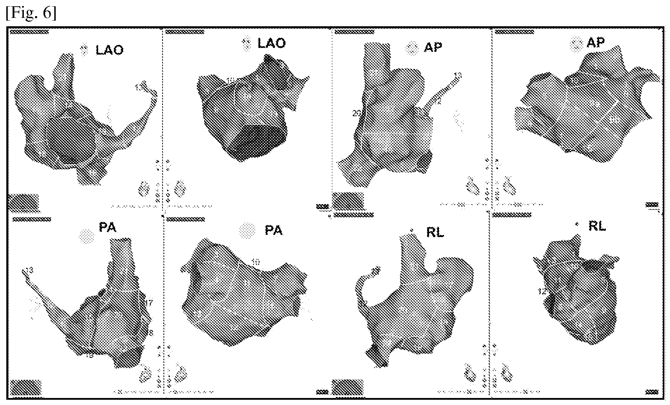

Pour tenter de classifier l'origine de la TA, la Demanderesse a longtemps travaillé sur la base d'une division des oreillettes en 21 zones comme représenté sur la [Fig.6]. Au cours de ses travaux, et à la recherche d'une solution pouvant être utilisée en temps réel, elle s'est rendu compte qu'il est pertinent de regrouper ces zones en 4 groupes : l'oreillette gauche (zones 7, 8, 9A, 9B, 10, 11A, 11B et 12), l'oreillette droite (zones 18, 19, 20B, 20H, et 22), le septum (zones 5, 6, 14, 15, 17 et 21), et la partie latérale de l'oreillette gauche (zones 1, 2, 3, 4, 13 et 16).

Comme on le verra plus bas, le dispositif de l'invention permet de déterminer, pour un ensemble de signaux ECG et d'un cathéter de référence intracardiaque communément placé dans la veine du sinus coronaire, que l'on désignera par l'expression "CS" dans la suite, de déterminer le groupe qui contient l'origine de la TA. Cette détermination est particulièrement intéressante car elle offre un point de départ au praticien pour chercher et déterminer la zone précise d'origine de la TA et donc la partie malade du cœur. En effet, même si ces groupes définissent 4 zones plus vastes que les 21 zones originelles, cela suffit déjà à procurer un grand avantage dans la procédure.

En outre, la combinaison de signaux ECG et de signaux tirés du sinus coronaire est particulièrement innovante. Elle n'a jamais été présentée précédemment, et elle permet l'utilisation d'un apprentissage automatique, ce qui permet le fonctionnement temps réel du dispositif 2.

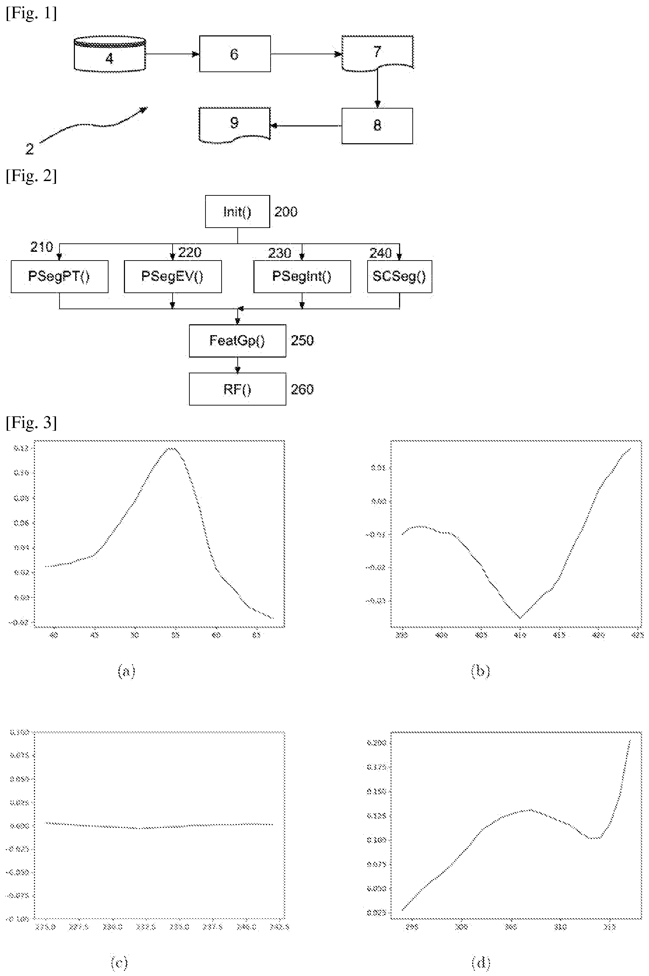

La [Fig.1] représente un diagramme schématique d'un dispositif de traitement de signaux cardiaques 2 selon l'invention. Comme on peut le voir sur cette figure, le dispositif 2 comprend une mémoire 4, un extracteur 6 et un localisateur 8. L'extracteur 6 détermine un ensemble de caractéristiques de jeu de données 7 qui sera décrit plus bas et qui sert d'entrée au localisateur 8 qui retourne une zone d'origine 9.

La mémoire 4 reçoit en entrée des jeux de données d'entrée. Chaque jeu de données d'entrée comprend une pluralité de segments d'onde P chacun associé à une piste d'électrocardiogramme et à une fenêtre temporelle d'acquisition, et une pluralité de segments tirés de signaux de sinus coronaire associés à la même fenêtre temporelle d'acquisition. Cela signifie concrètement qu'une fenêtre de temps, typiquement de 10 secondes avant un temps de mesure courant, est utilisée pour découper un ECG à 9 pistes (tirées d'un capteur 12 pistes dont 3 sont ignorées car correspondant à une combinaison linéaire de certaines des autres pistes) ainsi que 5 pistes de signaux CS. Dans le cas particulier de l'ECG, seuls les segments correspondant à une onde P sont utilisés. Comme chaque rythme cardiaque est particulier, cela signifie que le jeu de données d'entrée reçoit un nombre variable de segments d'onde P qui sont chacun tirés d'une piste d'ECG. La présente invention ne s'intéresse pas à la méthode particulière d'obtention des segments d'onde P et considère que ceux-ci forment une entrée. Dans certains modes de réalisation, le dispositif 2 pourrait être agencé pour analyser directement les pistes ECG et y déterminer les segments d'onde P. Par segment d'onde P, on entend que des sous-ensembles de chaque piste d'ECG sont découpés à l'intérieur de la fenêtre de temps, et définissent un segment de cette dernière.

Comme on le verra plus bas, les segments d'onde P de chaque jeu de données sont tous traités afin d'en tirer des caractéristiques *("features"* en anglais) qui servent d'entrée au localisateur 8. Ainsi, pour chaque piste d'ECG, 6 caractéristiques sont déterminées, tandis que 4 caractéristiques sont tirées des signaux CS. Cela fait donc un total de 58 caractéristiques (6 caractéristiques pour 9 pistes plus 4) qui forment le vecteur d'entrée du localisateur 8. En variante, le dispositif 2 pourrait déterminer que certains segments d'onde P ou signaux CS sont difficilement exploitables et ne pas tirer des caractéristiques pour chacun de ceux-ci mais en écarter certains. Toujours en variante, l'apprentissage automatique pourrait être fait sur un nombre moindre de caractéristiques, par exemple en ne déterminant qu'entre 3 et 5 caractéristiques par piste d'ECG. Enfin, le dispositif 2 pourrait également choisir de ne conserver que certaines pistes d'ECG (et donc les segments d'onde P correspondants), et réduire ainsi le nombre de caractéristiques.

La mémoire 4 peut être tout type de stockage de données propre à recevoir des données numériques : disque dur, disque dur à mémoire flash, mémoire flash sous toute forme, mémoire vive, disque magnétique, stockage distribué localement ou dans le cloud, etc.

Dans l'exemple décrit ici, la mémoire 4 reçoit toutes les données qui concernent le dispositif 2, c'est-à-dire les programmes et logiciels instanciant l'extracteur 6 et le localisateur 8, les paramètres et hyperparamètres de ceux-ci, les poids des arbres, les jeux de données reçues en entrée (le cas échéant), les caractéristiques déterminées par l'extracteur 6, les données stockées en mémoire tampon, ainsi que les données de zone d'origine en sortie. Les données calculées par le dispositif peuvent être stockées sur tout type de mémoire similaire à la mémoire 4, ou sur celle-ci. Ces données peuvent être effacées après que le dispositif a effectué ses tâches ou conservées.

L'extracteur 6 et le localisateur 8 accèdent directement ou indirectement à la mémoire 4. Ils peuvent être réalisés sous la forme d'un code informatique approprié exécuté sur un ou plusieurs processeurs. Par processeurs, il doit être compris tout processeur adapté aux calculs décrits plus bas. Un tel processeur peut être réalisé de toute manière connue, sous la forme d'un microprocesseur pour ordinateur personnel, portable, tablette ou smartphone, d'une puce dédiée de type FPGA ou SoC, d'une ressource de calcul sur une grille ou dans le cloud, d'une grappe de processeurs graphiques (GPUs), d'un microcontrôleur, ou de toute autre forme propre à fournir la puissance de calcul nécessaire à la réalisation décrite plus bas. Un ou plusieurs de ces éléments peuvent également être réalisés sous la forme de circuits électroniques spécialisés tel un ASIC. Une combinaison de processeur et de circuits électroniques peut également être envisagée. Des processeurs dédiés à l'apprentissage automatique pourront aussi être utilisés.

La [Fig.2] représente un exemple de mise en œuvre d'une fonction exécutée par l'extracteur 6 pour extraire l'ensemble de caractéristiques de jeu de données 7 et pour en tirer la zone d'origine 9.

Dans une opération 200, l'extracteur 6 met en œuvre une fonction Init() qui reçoit l'instant courant, et sélectionne dans la mémoire 4 les segments d'onde P qui correspondent à la fenêtre temporelle définie par l'instant courant. Ainsi, comme décrit plus haut, la fenêtre temporelle a pour extrémité l'instant courant d'une part, et un instant situé 10 secondes en amont de l'instant courant d'autre part. La Demanderesse a constaté que la durée de 10 secondes permet d'obtenir un nombre suffisant de segments d'onde P pour obtenir une zone d'origine fiable avec des instants courants pris toutes les 10 secondes. En variante, cette durée pourrait être différente, par exemple une durée de fenêtre temporelle de 2 secondes, 5 secondes, ou 15 secondes dès lors qu'au moins un battement cardiaque a été détecté en vue de l'obtention des ondes P.

Ainsi, la fonction Init() extrait des segments d'onde P et des signaux CS qui correspondent à une unique fenêtre temporelle. Ensuite, quatre fonctions sont exécutées en parallèle pour déterminer des données qui permettront de produire l'ensemble de caractéristiques de jeu de données 7.

Dans une opération 210, une fonction PSegPT() est exécutée pour déterminer, pour chaque segment d'onde P, un profil de polarité d'onde P lui correspondant. En effet, la Demanderesse a déterminé que le profil de polarité d'onde P est un indicateur significatif de la zone d'origine de la TA. Pour cela, la fonction PSegPT() met en œuvre un calcul de déformation temporelle dynamique (*"Dynamic Time Warping"* en anglais ou *"DTW"*) afin de déterminer la similarité entre chaque segment d'onde P et chacun parmi 4 profils de polarité d'onde P que la Demanderesse a identifiés comme discriminants pour déterminer la zone d'origine de la TA. La [Fig.3] représente un exemple de chacun de ces 4 profils.

Pour rappel, la déformation temporelle dynamique est un algorithme permettant de mesurer la similarité entre deux suites de valeurs qui varient au cours du temps. Les suites de valeurs sont déformées par transformation non linéaire de la variable temporelle pour déterminer une mesure de leur similarité indépendamment de certaines transformations non linéaires du temps. Ainsi, en comparant les mesures de DTW entre un segment d'onde P donné et les 4 profils de polarité d'onde P, ont peut retenir que le profil de polarité d'onde P pour un segment d'onde P donné est celui dont la DTW entre les deux est la plus faible. Cela revient à considérer que ce profil de polarité d'onde P est celui auquel le segment d'onde P donné "ressemble le plus". En variante, si aucun profil de polarité d'onde P ne paraît être le plus pertinent, un type "non déterminé" pourrait être retenu afin d'améliorer l'apprentissage automatique. En variante, la DTW pourrait être remplacée par la distance euclidienne, ou une autre distance au sens mathématique à minimiser entre l'onde P et les 4 profils. On pourrait également utiliser une autre mesure de corrélation avec les profils.

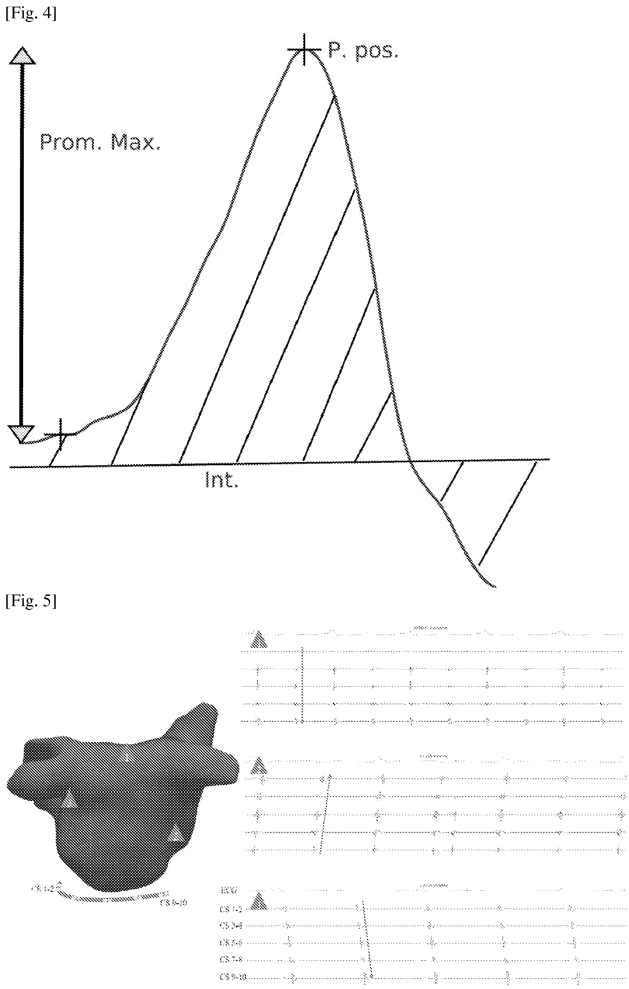

Dans une opération 220, une fonction PSegEV() est exécutée. Cette fonction a pour rôle d'extraire pour chaque segment d'onde P des valeurs qui caractérisent ses extrema (*"peak"* en anglais). Ainsi, comme représenté sur la [Fig.4], les valeurs d'extremum pertinentes qui peuvent être retenues sont le nombre d'extrema positifs, le nombre d'extrema négatifs, la prominence positive maximale, et la prominence négative maximale. Ces quatre caractéristiques permettent de définir une image de chaque segment d'onde P, à savoir son nombre de pics en positif comme en négatif (à quel point le segment d'onde P est-il chaotique), ainsi que l'intensité de ces pics (en géographie, la prominence est la différence d'altitude entre un sommet donné et l'ensellement ou le col le plus élevé permettant d'atteindre une cime encore plus haute, et cette notion est naturellement étendue en analyse graphique de signal). En fonction des situations, comme décrit plus haut, seul un type ou deux types de caractéristiques pourraient être calculés, c'est-à-dire par exemple seul le nombre de pics positifs, ou en complément la prominence positive maximale. De plus, certains segments d'onde P pourraient être écartés si leur forme se prête mal à ces calculs.

Dans une opération 230, une fonction PSegInt() est exécutée. Cette fonction a pour rôle de calculer pour chaque segment d'onde P l'intégrale du signal correspondant. En effet, cette intégrale caractérise également la force mesurée de l'onde P. Ici encore, certains segments d'onde P pourraient être écartés si cela s'avère pertinent. En variante, deux valeurs d'intégrale peuvent être calculées : l'intégrale de la partie positive et l'intégrale de la partie négative, qui permettent ensemble de mieux décrire encore le signal. Dans ce cas, l'ensemble de caractéristiques de jeu de données contient plutôt 67 entrées (58 valeurs plus 9 valeurs d'intégrale en plus).

Enfin, dans une opération 240, une fonction SCSeg() est exécutée. Cette fonction a pour rôle de déterminer les instants d'activation au sein de chaque piste de sinus coronaire, et de retourner des valeurs de temps indiquant les décalages entre les instants de début d'activation. En effet, la séquence des pics dans les signaux CS peut indiquer si la zone d'origine d'AT se trouve dans l'oreillette gauche ou droite, et la Demanderesse a découvert que ces caractéristiques se combinent particulièrement bien avec les caractéristiques tirées des segments d'onde P dans le cadre d'un apprentissage automatique. La [Fig.5] illustre un exemple de signaux CS acquis en fonction de la localisation de la TA en des emplacements indiqués par un triangle muni d'un indice 1, 2 ou 3, sur l'oreillette gauche, ainsi que les séquences d'activation correspondantes (flèches sur chaque acquisition). Dans l'exemple décrit ici, les valeurs de temps sont toutes prises en référence à l'instant d'activation du premier signal de sinus coronaire. En variante, ces valeurs pourraient être entre deux instants d'activation de signaux successifs (par exemple différence entre le premier et le deuxième signal, le deuxième et le troisième signal, le troisième et le quatrième signal, et le quatrième et le cinquième signal). L'important est que les valeurs de temps mesurées soient représentatives de la séquence des pics dans les signaux CS.

Bien que les opérations 210, 220 et 230 aient été présentées séparément comme exécutées par trois fonctions distinctes, elles pourraient être exécutées de manière séquentielle ou parallèle au sein d'une unique fonction. De même, l'opération 240 pourrait être réalisée séquentiellement par rapport aux opérations 210 à 230, et non pas en parallèle. La parallélisation de ces opérations présente cependant l'avantage d'être plus rapide et donc de favoriser l'exécution en temps réel.

Un problème se pose cependant en vue de l'exécution d'un calcul par apprentissage automatique : comme on l'a vu plus haut, comme c'est l'instant de mesure (l'instant courant) qui détermine la fenêtre temporelle, le nombre de segments d'onde P n'est pas constant lors de l'exécution du dispositif 2. Au-delà de cela, il y a même un risque de sur-représentation de segments d'onde P associés à une piste d'ECG particulière par rapport aux autres. Enfin, si le nombre de segments d'onde P n'est pas constant, il n'est pas possible de définir un vecteur d'entrée de dimension fixe pour l'apprentissage automatique.

Par conséquent, la fonction de la [Fig.2] se poursuit par une opération 250 dans laquelle une fonction FeatGp() est exécutée par l'extracteur 6. Cette fonction vient dans un premier temps regrouper toutes les données calculées par les opérations 210 à 230 en fonction de la piste d'ECG dont sont tirés les segments d'onde P à l'origine de ces valeurs. Cela signifie que tous les profils de polarité d'onde P, les valeurs d'extremum et les valeurs d'intégrale associées à une même piste d'ECG sont regroupés dans un groupe de données d'onde P de piste. Ensuite, dans chaque groupe de données d'onde P de piste, une unique valeur est retenue pour chaque type de caractéristique, c'est-à-dire un profil de polarité d'onde P, une valeur d'extremum pour chaque type ayant fait l'objet d'un calcul, et une valeur d'intégrale. Comme mentionné précédemment, de manière préférée, il a quatre valeurs d'extremum correspondant respectivement aux nombres de pics (positifs et négatifs) et à la prominence maximale (positive et négative). Dans le cas des profils de polarité d'onde P, un vote par majorité peut être effectué : pour une piste d'ECG donné, c'est le plus grand nombre de profils de polarité d'onde P des segments d'onde P qui désigne le profil retenu. Dans le cas des valeurs d'extremum et d'intégrale, c'est la moyenne qui peut être utilisée. D'autres méthodes pourront être utilisées : moyenne pondérée, médiane, régression, et seuil minimum pour le vote par majorité.

Ainsi, 6 caractéristiques sont obtenues pour chaque piste d'ECG, et elles forment des ensembles de caractéristiques d'onde P de piste. En groupant ces ensembles avec le jeu de valeurs de temps tirées des signaux CS, on obtient l'ensemble de caractéristiques de jeu de données comprenant les 58 caractéristiques mentionnées plus haut. Lorsque les signaux CS contiennent plusieurs séquences d'activation, les jeux de valeurs pourront être moyennés dans l'opération 250 de manière similaire aux valeurs d'extremum des segments d'onde P.

Cet ensemble peut alors être transmis comme vecteur d'entrée au localisateur 8 qui exécute une fonction RF() dans une opération 260. La fonction RF() met en œuvre un classificateur à forêts aléatoires qui a été entraîné avec un jeu de données d'entraînement dans lequel la zone d'origine en sortie était connue. Ainsi, grâce à l'entraînement du moteur d'apprentissage automatique, la zone d'origine 9 peut être déterminée en temps réel.

En variante, le localisateur 8 pourrait mettre en œuvre un apprentissage automatique utilisant des arbres décisionnels différents des forêts aléatoires, par exemple basé sur le renforcement du gradient (*"gradient boosting"* anglais), ou le renforcement du gradient extrême (*"XGBoost"* en anglais).

Les tests de la Demanderesse ont permis de démontrer que le dispositif 2 permet d'obtenir des résultats très satisfaisants, même avec une base d'entraînement assez restreinte. Dans cette base d'entraînement, les signaux d'ECG et CS étaient associés à des zones d'origine de TA selon la répartition suivante : Oreillette gauche (47), Oreillette droite (7), Septum (158), Latérale (24). Ainsi, les résultats obtenus avec cette base d'entraînement sont résumés dans le tableau ci-dessous :

| Groupe de zone | Précision | Rappel | Nombre dans l'ensemble de test |
|---|---|---|---|
| Oreillette gauche | 92% | 80% | 15 |
| Oreillette droite | 0 | 0 | 3 |
| Septum | 68% | 94% | 18 |
| Latérale | 0 | 0 | 3 |

Le score Kappa de Cohen a été mesuré à 0,55, ce qui est très favorable, et le taux de prédiction global est de 74%. Pour rappel, le score Kappa de Cohen mesure l'accord entre les annotations du jeu de test et la prédiction qui en est faite, en prenant en compte le déséquilibre dans la représentation des classes à l'intérieur de celui-ci.

Ainsi, le dispositif 2 permet de réaliser une détermination "haut niveau" de la zone d'origine d'une AT en temps réel, ce qui permet d'accélérer considérablement les procédures d'ablation dans le cadre du traitement de la TA, et diminue les risques d'erreur liés à l'exploration de zone "inutiles" du cœur, c'est-à-dire non susceptibles d'être à l'origine de la TA.

## Revendications

1. Dispositif de traitement de signaux cardiaques comprenant
- une mémoire (4) agencée pour recevoir des jeux de données d'entrée
comprenant chacun une pluralité de segments d'onde P chacun associé à une piste d'électrocardiogramme et à une fenêtre temporelle
d'acquisition, et une pluralité de signaux de sinus coronaire associés à la même fenêtre temporelle d'acquisition et présentant une ou plusieurs séquences d'activation,
- un extracteur (6) agencé, pour un jeu de données d'entrée donné, pour déterminer pour certains au moins des segments d'onde P du jeu de
données d'entrée donné :
* un type de profil de polarité d'onde,
* au moins une caractéristique d'extremum d'un type choisi dans un groupe de types comprenant le nombre d'extrema locaux positifs, le nombre d'extrema locaux négatifs, le maximum de prominence positive et le maximum de prominence négative, et
* au moins une valeur d'intégrale de ces segments d'onde P,
et pour associer les données résultantes en groupe de données d'onde P de piste en fonction de la piste d'électrocardiogramme à laquelle est associé chaque segment d'onde P à partir duquel lesdites données résultantes ont été calculées,
l'extracteur (6) étant en outre agencé pour déterminer, pour chaque groupe de données d'onde P de piste, un ensemble de caractéristiques d'onde P de piste comprenant un type de profil de polarité, une valeur de caractéristique d'extremum de jeu de données pour chaque type de caractéristique d'extremum calculé et une valeur d'intégrale déterminés à partir des données du groupe de données d'onde P de piste correspondant,
l'extracteur (6) étant en outre agencé pour déterminer des temps d'activation dans certaines au moins des séquences d'activation des signaux de sinus coronaire du jeu de données d'entrée donné et pour en tirer un jeu de valeurs de temps, et pour retourner un ensemble de caractéristiques de jeu de données comprenant d'une part le jeu de valeurs de temps, et d'autre part les ensembles de caractéristiques d'onde P de piste, et
- un localisateur (8) à base d'apprentissage automatique utilisant des arbres décisionnels agencé pour recevoir en entrée un ensemble de caractéristiques de jeu de données, et pour retourner en sortie un identifiant de région cardiaque.

2. Dispositif selon la revendication 1, dans lequel le localisateur (8) est agencé pour mettre en œuvre un classificateur à forêts aléatoires.

3. Dispositif selon la revendication 1 ou 2, dans lequel l'extracteur (6) est agencé pour déterminer une valeur de caractéristique d'extremum de jeu de données indiquant une absence de détermination pour un type de caractéristique d'extremum non calculé, et pour retourner un ensemble de caractéristiques de jeu de données comprenant une valeur de caractéristique d'extremum de jeu de données pour chaque type de caractéristique d'extremum.

4. Dispositif selon la revendication 1 ou 2, dans lequel l'extracteur (6) est agencé pour déterminer une valeur de caractéristique d'extremum de jeu de données pour chaque type du groupe de types.

5. Dispositif selon l'une des revendications précédentes, dans lequel l'extracteur (6) est agencé, pour un ensemble de caractéristiques d'onde P de piste donné, pour déterminer le type de profil de polarité en retenant le type de profil de polarité d'onde majoritaire dans le groupe de données d'onde P de piste correspondant.

6. Dispositif selon l'une des revendications précédentes, dans lequel l'extracteur (6) est agencé, pour un ensemble de caractéristiques d'onde P de piste donné, pour déterminer la valeur de caractéristique d'extremum de jeu de données pour chaque type de caractéristique d'extremum calculé et la valeur d'intégrale à partir de la moyenne de ces valeurs dans le groupe de données d'onde P de piste correspondant.

7. Dispositif selon l'une des revendications précédentes, dans lequel l'extracteur (6) est agencé pour calculer le jeu de valeurs de temps à partir de la différence entre les temps d'activation des séquences d'activation des signaux de sinus coronaire.

8. Dispositif selon l'une des revendications précédentes, dans lequel le localisateur (8) est agencé pour recevoir en entrée un ensemble de caractéristiques de jeu de données comprenant 9 ensembles de caractéristiques d'onde P de piste, et un jeu de valeurs de temps comprenant 4 valeurs.

9. Procédé de traitement de signaux cardiaques mis en œuvre par ordinateur comprenant les opérations suivantes :
a) recevoir des jeux de données d'entrée comprenant chacun une pluralité de segments d'onde P chacun associé à une piste d'électrocardiogramme et à une fenêtre temporelle d'acquisition, et une pluralité de signaux de sinus coronaire associés à la même fenêtre temporelle d'acquisition et présentant une ou plusieurs séquences d'activation,
b) pour un jeu de données d'entrée donné, pour certains au moins des segments d'onde P du jeu de données d'entrée donné :
b1) déterminer (210) un type de profil de polarité d'onde,
b2) déterminer (220) au moins une caractéristique d'extremum d'un type choisi dans un groupe de types comprenant le nombre d'extrema locaux positifs, le nombre d'extrema locaux négatifs, le maximum de prominence positive et le maximum de prominence négative, et
b3) déterminer (230) au moins une valeur d'intégrale de ces segments d'onde P,
b4) associer (250) les données résultantes des opérations b1) à b3) en groupe de données d'onde P de piste en fonction de la piste d'électrocardiogramme à laquelle est associé chaque segment d'onde P à partir duquel lesdites données résultantes ont été calculées,
b5) déterminer (250), pour chaque groupe de données d'onde P de piste, un ensemble de caractéristiques d'onde P de piste comprenant un type de profil de polarité, une valeur de caractéristique d'extremum de jeu de données pour chaque type de caractéristique d'extremum calculé et une valeur d'intégrale déterminés à partir des données du groupe de données d'onde P de piste correspondant,
b6) déterminer (240) des temps d'activation dans certaines au moins des séquences d'activation des signaux de sinus coronaire du jeu de données d'entrée donné et pour en tirer un jeu de valeurs de temps,
b7) retourner (250) un ensemble de caractéristiques de jeu de données comprenant d'une part le jeu de valeurs de temps de l'opération b6), et d'autre part les ensembles de caractéristiques d'onde P de piste de l'opération b5), et
c) fournir (260) un ensemble de caractéristiques de jeu de données obtenu à l'opération b7) en entrée d'un localisateur (8) à base d'apprentissage automatique utilisant des arbres décisionnels, et retourner en sortie un identifiant de région cardiaque.

10. Procédé selon la revendication 9, dans lequel l'opération b2) comprend déterminer une valeur de caractéristique d'extremum de jeu de données pour chaque type du groupe de types.

11. Procédé selon la revendication 9 ou 10, dans lequel l'opération b5) comprend, pour un ensemble de caractéristiques d'onde P de piste donné, déterminer le type de profil de polarité en retenant le type de profil de polarité d'onde majoritaire dans le groupe de données d'onde P de piste correspondant.

12. Procédé selon l'une des revendications 9 à 11, dans lequel l'opération b5) comprend, pour un ensemble de caractéristiques d'onde P de piste donné, déterminer la valeur de caractéristique d'extremum de jeu de données pour chaque type de caractéristique d'extremum calculé et la valeur d'intégrale à partir de la moyenne de ces valeurs dans le groupe de données d'onde P de piste correspondant.

13. Procédé selon l'une des revendications 9 à 12, dans lequel l'opération b6) comprend calculer le jeu de valeurs de temps à partir de la différence entre les temps d'activation des séquences d'activation des signaux de sinus coronaire.

14. Programme informatique comprenant des instructions pour exécuter le procédé selon l'une des revendications 9 à 13 lorsque ledit programme informatique est mis en œuvre par ordinateur.

15. Support de stockage de données sur lequel est enregistré le programme informatique selon la revendication 14.

## Patentansprüche

1. Vorrichtung zur Verarbeitung von Herzsignalen, umfassend
• einen Speicher (4), der eingerichtet ist, um Eingangsdatensätze zu empfangen, die jeweils eine Vielzahl von P-Wellen-Segmenten umfassen, die jeweils einer Elektrokardiogramm-Ableitung und einem Erfassungszeitfenster zugeordnet sind, und eine Vielzahl von Koronarsinussignalen, die demselben Erfassungszeitfenster zugeordnet sind und eine oder mehrere Aktivierungssequenzen aufweisen,
• einen Extraktor (6), der eingerichtet ist, um für einen gegebenen Eingangsdatensatz für mindestens einige der P-Wellen-Segmente des gegebenen Eingangsdatensatzes Folgendes zu bestimmen:
• einen Wellenpolaritätsprofiltyp,
• mindestens ein Extremum-Merkmal eines Typs, der aus einer Gruppe von Typen ausgewählt ist, die die Anzahl positiver lokaler Extrema, die Anzahl negativer lokaler Extrema, die maximal positive Prominenz und die maximal negative Prominenz umfasst, und
• mindestens einen Integralwert dieser P-Wellen-Segmente,
• wobei der Extraktor (6) ferner eingerichtet ist, um für jede Ableitungs-P-Wellen-Datensatzgruppe einen Ableitungs-P-Wellen-Merkmalsatz zu bestimmen, der einen Polaritätsprofiltyp, einen Datensatz-Extremum-Merkmalwert für jeden berechneten Extremum-Merkmalstyp und einen Integralwert umfasst, die aus den Daten der entsprechenden Ableitungs-P-Wellen-Datensatzgruppe bestimmt wurden,
• wobei der Extraktor (6) ferner eingerichtet ist, um Aktivierungszeiten in mindestens einigen der Aktivierungssequenzen der Koronarsinussignale des gegebenen Eingangsdatensatzes zu bestimmen und daraus einen Satz von Zeitwerten abzuleiten, und um einen Datensatz-Merkmalsatz zurückzugeben, der einerseits den Satz von Zeitwerten und andererseits die Ableitungs-P-Wellen-Merkmalsätze umfasst, und
• einen auf maschinellem Lernen basierenden Lokalisierer (8), der Entscheidungsbäume verwendet und eingerichtet ist, um als Eingabe einen Datensatz-Merkmalsatz zu empfangen und als Ausgabe einen Herzregionsidentifikator zurückzugeben.

2. Vorrichtung nach Anspruch 1, wobei der Lokalisierer (8) eingerichtet ist, um einen Random-Forest-Klassifizierer zu implementieren.

3. Vorrichtung nach Anspruch 1 oder 2, wobei der Extraktor (6) eingerichtet ist, um einen Datensatz-Extremum-Merkmalwert zu bestimmen, der ein Fehlen einer Bestimmung für einen nicht berechneten Extremum-Merkmalstyp angibt, und um einen Datensatz-Merkmalsatz zurückzugeben, der einen Datensatz-Extremum-Merkmalwert für jeden Extremum-Merkmalstyp umfasst.

4. Vorrichtung nach Anspruch 1 oder 2, wobei der Extraktor (6) eingerichtet ist, um einen Datensatz-Extremum-Merkmalwert für jeden Typ der Gruppe von Typen zu bestimmen.

5. Vorrichtung nach einem der vorhergehenden Ansprüche, wobei der Extraktor (6) eingerichtet ist, um für einen gegebenen Ableitungs-P-Wellen-Merkmalsatz den Polaritätsprofiltyp zu bestimmen, indem der Mehrheits-Wellenpolaritätsprofiltyp in der entsprechenden Ableitungs-P-Wellen-Datensatzgruppe ausgewählt wird.

6. Vorrichtung nach einem der vorhergehenden Ansprüche, wobei der Extraktor (6) eingerichtet ist, um für einen gegebenen Ableitungs-P-Wellen-Merkmalsatz den Datensatz-Extremum-Merkmalwert für jeden berechneten Extremum-Merkmalstyp und den Integralwert aus dem Mittelwert dieser Werte in der entsprechenden Ableitungs-P-Wellen-Datensatzgruppe zu bestimmen.

7. Vorrichtung nach einem der vorhergehenden Ansprüche, wobei der Extraktor (6) eingerichtet ist, um den Satz von Zeitwerten aus der Differenz zwischen den Aktivierungszeiten der Aktivierungssequenzen der Koronarsinussignale zu berechnen.

8. Vorrichtung nach einem der vorhergehenden Ansprüche, wobei der Lokalisierer (8) eingerichtet ist, um als Eingabe einen Datensatz-Merkmalsatz zu empfangen, der 9 Ableitungs-P-Wellen-Merkmalsätze umfasst, und einen Satz von Zeitwerten, der 4 Werte umfasst.

9. Computerimplementiertes Verfahren zur Verarbeitung von Herzsignalen, umfassend die folgenden Schritte:
• a) Empfangen von Eingangsdatensätzen, die jeweils eine Vielzahl von P-Wellen-Segmenten umfassen, die jeweils einer Elektrokardiogramm-Ableitung und einem Erfassungszeitfenster zugeordnet sind, und eine Vielzahl von Koronarsinussignalen, die demselben Erfassungszeitfenster zugeordnet sind und eine oder mehrere Aktivierungssequenzen aufweisen,
• b) für einen gegebenen Eingangsdatensatz für mindestens einige der P-Wellen-Segmente des gegebenen Eingangsdatensatzes:
• b1) Bestimmen (210) eines Wellenpolaritätsprofiltyps,
• b2) Bestimmen (220) mindestens eines Extremum-Merkmals eines Typs, der aus einer Gruppe von Typen ausgewählt ist, die die Anzahl positiver lokaler Extrema, die Anzahl negativer lokaler Extrema, die maximal positive Prominenz und die maximal negative Prominenz umfasst, und
• b3) Bestimmen (230) mindestens eines Integralwerts dieser P-Wellen-Segmente,
• b4) Zuordnen (250) der aus den Schritten b1) bis b3) resultierenden Daten in eine Ableitungs-P-Wellen-Datensatzgruppe in Abhängigkeit von der Elektrokardiogramm-Ableitung, der jedes P-Wellen-Segment zugeordnet ist, von dem aus die resultierenden Daten berechnet wurden,
• b5) Bestimmen (250) für jede Ableitungs-P-Wellen-Datensatzgruppe eines Ableitungs-P-Wellen-Merkmalsatzes, der einen Polaritätsprofiltyp, einen Datensatz-Extremum-Merkmalwert für jeden berechneten Extremum-Merkmalstyp und einen Integralwert umfasst, die aus den Daten der entsprechenden Ableitungs-P-Wellen-Datensatzgruppe bestimmt wurden,
• b6) Bestimmen (240) von Aktivierungszeiten in mindestens einigen der Aktivierungssequenzen der Koronarsinussignale des gegebenen Eingangsdatensatzes und Ableiten eines Satzes von Zeitwerten daraus,
• b7) Zurückgeben (250) eines Datensatz-Merkmalsatzes, der einerseits den Satz von Zeitwerten aus Schritt b6) und andererseits die Ableitungs-P-Wellen-Merkmalsätze aus Schritt b5) umfasst, und
• c) Bereitstellen (260) eines in Schritt b7) erhaltenen Datensatz-Merkmalsatzes als Eingabe für einen auf maschinellem Lernen basierenden Lokalisierer (8), der Entscheidungsbäume verwendet, und Zurückgeben eines Herzregionsidentifikators als Ausgabe.

10. Verfahren nach Anspruch 9, wobei Schritt b2) das Bestimmen eines Datensatz-Extremum-Merkmalwerts für jeden Typ der Gruppe von Typen umfasst.

11. Verfahren nach Anspruch 9 oder 10, wobei Schritt b5) für einen gegebenen Ableitungs-P-Wellen-Merkmalsatz das Bestimmen des Polaritätsprofiltyps umfasst, indem der Mehrheits-Wellenpolaritätsprofiltyp in der entsprechenden Ableitungs-P-Wellen-Datensatzgruppe ausgewählt wird.

12. Verfahren nach einem der Ansprüche 9 bis 11, wobei Schritt b5) für einen gegebenen Ableitungs-P-Wellen-Merkmalsatz das Bestimmen des Datensatz-Extremum-Merkmalwerts für jeden berechneten Extremum-Merkmalstyp und des Integralwerts aus dem Mittelwert dieser Werte in der entsprechenden Ableitungs-P-Wellen-Datensatzgruppe umfasst.

13. Verfahren nach einem der Ansprüche 9 bis 12, wobei Schritt b6) das Berechnen des Satzes von Zeitwerten aus der Differenz zwischen den Aktivierungszeiten der Aktivierungssequenzen der Koronarsinussignale umfasst.

14. Computerprogramm, umfassend Anweisungen zur Ausführung des Verfahrens nach einem der Ansprüche 9 bis 13, wenn das Computerprogramm durch einen Computer ausgeführt wird.

15. Datenspeichermedium, auf dem das Computerprogramm nach Anspruch 14 aufgezeichnet ist.

## Claims

1. A cardiac signal processing device comprising
- a memory (4) configured to receive sets of input data, each comprising a plurality of P-wave segments, each associated with an electrocardiogram lead and an acquisition time window, and a plurality of coronary sinus signals associated with the same acquisition time window and exhibiting one or more activation sequences,
- an extractor (6) configured, for a given set of input data, to determine, for at least some of the P-wave segments in the given set of input data :
* a type of wave polarity profile,
* at least one extremum feature of a type selected from a group of types comprising the number of positive local extrema, the number of negative local extrema, the maximum positive prominence, and the maximum negative prominence, and
* at least one integral value of these P-wave segments,
and for grouping the resulting data into P-wave data groups by lead based on the electrocardiogram lead associated with each P-wave segment from which said resulting data were calculated,
the extractor (6) being further configured to determine, for each group of P-wave data per lead, a set of P-wave characteristics per lead comprising a polarity profile type, a dataset extremum feature value for each type of extremum feature calculated, and an integral value determined from the data of the corresponding group of P-wave data per lead,
the extractor (6) being further configured to determine activation times in at least some of the activation sequences of the coronary sinus signals in the given input dataset and to derive a set of time values therefrom, and to return a set of dataset features comprising, on the one hand, the set of time values, and, on the other hand, the sets of P-wave track features, and
- a locator (8) based on machine learning using decision trees configured to receive as input a set of dataset features, and to return as output a cardiac region identifier.

2. A device according to claim 1, wherein the locator (8) is configured to implement a random forest classifier.

3. A device according to claim 1 or 2, wherein the extractor (6) is configured to determine a dataset extremum feature value indicating an undetermined result for a type of extremum feature that has not been calculated, and to return a set of dataset features comprising a dataset extremum feature value for each type of extremum feature.

4. A device according to claim 1 or 2, wherein the extractor (6) is configured to determine a dataset extremum feature value for each type in the group of types.

5. A device according to one of the preceding claims, wherein the extractor (6) is configured, for a given set of track P-wave features, to determine the polarity profile type by retaining the majority P-wave polarity profile type in the corresponding track P-wave data set.

6. A device according to one of the preceding claims, wherein the extractor (6) is configured, for a given set of track P-wave characteristics, to determine the data set extremum feature value for each type of calculated extremum feature and the integral value based on the average of these values in the corresponding track P-wave data set.

7. A device according to one of the preceding claims, wherein the extractor (6) is configured to calculate the set of time values based on the difference between the activation times of the activation sequences of the coronary sinus signals.

8. A device according to one of the preceding claims, wherein the locator (8) is configured to receive as input a set of data-set features comprising 9 sets of P-wave track features, and a set of time values comprising 4 values.

9. A computer-implemented method for processing cardiac signals, comprising the following steps:
a) receiving input datasets, each comprising a plurality of P-wave segments, each associated with an electrocardiogram lead and an acquisition time window, and a plurality of coronary sinus signals associated with the same acquisition time window and exhibiting one or more activation sequences,
b) for a given input data set, for at least some of the P-wave segments in the given input data set:
b1) determining (210) a type of wave polarity profile,
b2) determine (220) at least one extremum feature of a type selected from a group of types comprising the number of positive local extrema, the number of negative local extrema, the maximum positive prominence, and the maximum negative prominence, and
b3) determining (230) at least one integral value of these P-wave segments,
b4) grouping (250) the data resulting from operations b1) through b3) into P-wave data groups by lead, based on the electrocardiogram lead associated with each P-wave segment from which said resulting data were calculated,
b5) determining (250), for each group of P-wave data per lead, a set of P-wave characteristics per lead comprising a polarity profile type, a dataset extremum feature value for each type of extremum feature calculated, and an integral value determined from the data in the corresponding group of P-wave data per lead,
b6) determining (240) activation times in at least some of the activation sequences of the coronary sinus signals in the given input dataset and deriving a set of time values therefrom,
b7) returning (250) a set of dataset features comprising, on the one hand, the set of time values from step b6), and, on the other hand, the sets of P-wave track features from step b5), and
c) providing (260) a set of dataset features obtained in step b7) as input to a machine learning-based locator (8) using decision trees, and outputting a cardiac region identifier.

10. A method according to claim 9, wherein step b2) comprises determining a data set extremum feature value for each type in the group of types.

11. A method according to claim 9 or 10, wherein step b5) comprises, for a given set of P-wave track features, determining the polarity profile type by selecting the majority P-wave polarity profile type in the corresponding P-wave track data set.

12. A method according to one of claims 9 through 11, wherein step b5) comprises, for a given set of track P-wave characteristics, determining the data set extremum feature value for each type of calculated extremum feature and the integral value based on the average of these values in the corresponding track P-wave data set.

13. A method according to one of claims 9 through 12, wherein step b6) comprises calculating the set of time values based on the difference between the activation times of the activation sequences of the coronary sinus signals.

14. A computer program comprising instructions for executing the method according to one of claims 9 through 13.

15. A data storage medium on which the computer program according to claim 14 is recorded.
